# EUROPÄISCHE PATENTANMELDUNG

(11) **EP 0 588 156 A2**
(43) Veröffentlichungstag der Anmeldung: **23.03.1994**
(21) Anmeldenummer: 93114013.1
(22) Anmeldetag: 02.09.1993
(51) Int. Cl.: C07C 209/16, C07C 211/03

(54) **Verfahren zur Herstellung von sekundären Aminen**

(30) Priorität: 11.09.1992 DE 4230402
(71) Anmelder: BASF Aktiengesellschaft, D-67063 Ludwigshafen (DE)
(72) Erfinder: Mueller, Herbert, Dr., D-6710 Frankenthal (DE)

(57) **Zusammenfassung**

Verfahren zur Herstellung von Dialkylaminen der allgemeinen Formel I 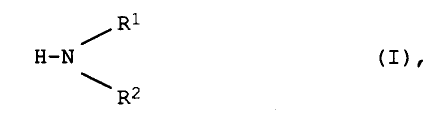
in der R¹, R² C₁- bis C₁₆-Alkyl bedeuten, durch Umsetzung von Monoalkylaminen der allgemeinen Formel II 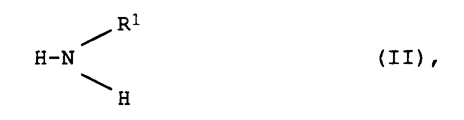
in der R¹ C₁- bis C₁₆-Alkyl bedeutet, mit Alkoholen R²-OH in Gegenwart eines Kupferchromit-Erdalkalichromit enthaltenden Hydrierungs-/Dehydrierungskatalysators, indem man die Umsetzung in flüssiger Phase im Molverhältnis Amin zum Alkohol von 1,5 : 1 bis 50 : 1 in Anwesenheit des bei der Reaktion gebildeten Wassers bei Temperaturen von 180 bis 210°C und Drücken von 40 bis 120 bar durchführt.

## Beschreibung

Die vorliegende Erfindung betrifft ein Verfahren zur Herstellung sekundärer Amine durch Umsetzung von primären Aminen mit Alkoholen in Gegenwart von Kupferchromit-Bariumchromit enthaltenden Hydrierungs-/Dehydrierungskatalysatoren bei erhöhten Temperaturen und Drücken.

Aus der DE-A-14 93 781 ist ein Verfahren zur Herstellung von sekundären Aminen bekannt, bei dem mindestens stöchiometrische Mengen eines primären Amins mit einem Alkohol umgesetzt werden. Ein Überschuß an Alkohol führt danach zu schlechten Selektivitäten von sekundären Aminen. Molverhältnisse Amin : Alkohol < 1 führen zu unerwünschten Kondensationsprodukten, die die Ausbeute drastisch vermindern und die Endprodukte stark verunreinigen.

Aus der DE-A-26 45 712 ist die Alkylierung von Ammoniak mit Alkoholen bekannt, wobei Ammoniak nur in dem Maße der Umsetzung zuzuführen ist, wie das entstehende Reaktionswasser aus dem Reaktionsmilieu entfernt werden kann. Ein solches Verfahren ist aufwendig und für höhere Alkohole nicht anwendbar. Selektivitäten von über 85 Mol-% erreicht man nur mit Alkoholen mit der C-Zahl < 7.

Aus der US-A-4 480 131 ist ein Verfahren zur Herstellung sekundärer Amine aus primären Alkylaminen und Alkoholen bei Temperaturen von 50 bis 250°C in Gegenwart von alkalisch behandelten Katalysatoren oder mit binären Katalysatorsystemen von Cu/ZnO und Pd/SiO2 bekannt. Diese schwierig herzustellenden Katalysatoren erlauben es nicht, Selektivitäten > 80 Mol-% zu erreichen.

Aus der DE-A-22 55 701 ist ein Verfahren zur Herstellung von sekundären Aminen durch Umsetzung von primären Alkoholen mit Ammoniak an suspendierten Hydrier-/Dehydrierkatalysatoren in flüssiger Phase bekannt, wobei - bei Normaldruck oder leicht erhöhtem Druck - bevorzugt bei Temperaturen oberhalb 160°C - definierte Ammoniak/Wasserstoffgas-Geschwindigkeiten eingehalten werden sollen. Mit Hilfe der im Überschuß angebotenen Gase wird gleichzeitig das entstehende Reaktionswasser aus dem Reaktionsgemisch abgeführt. Die beschriebene Verfahrensweise erfordert einen erheblichen apparativen Aufwand und sorgfältige Überwachung, z.B. ist stets dafür Sorge zu tragen, daß eine Mindestmenge an Ammoniak nicht unterschritten wird, weil sonst der Anteil an tertiärem Amin ansteigt.

Der vorliegenden Erfindung lag daher die Aufgabe zugrunde, den zuvor genannten Nachteilen abzuhelfen.

Demgemäß wurde ein neues und verbessertes Verfahren zur Herstellung von Dialkylaminen der allgemeinen Formel I 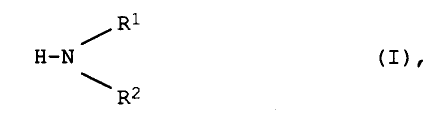
in der R¹, R² C₁- bis C₁₆-Alkyl bedeuten, durch Umsetzung von Monoalkylaminen der allgemeinen Formel II 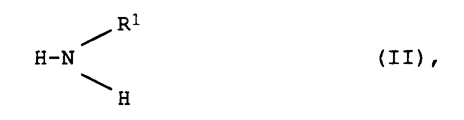
in der R¹ C₁- bis C₁₆-Alkyl bedeutet, mit Alkoholen R²-OH in Gegenwart eines Kupferchromit-Erdalkalichromit enthaltenden Hydrierungs-/Dehydrierungskatalysators gefunden, welches dadurch gekennzeichnet ist, daß man die Umsetzung in flüssiger Phase im Molverhältnis Amin zum Alkohol von 1,5 : 1 bis 50 : 1 in Anwesenheit des bei der Reaktion gebildeten Wassers bei Temperaturen von 180 bis 210°C und Drücken von 40 bis 120 bar durchführt.

Das erfindungsgemäße Verfahren kann wie folgt durchgeführt werden:
Die primären Amine II können mit einem Alkohol R²-OH bei Temperaturen von 160 bis 250°C, bevorzugt 170 bis 210°C im allgemeinen unter Überdruck wie z.B. unter einem Gesamtdruck von 40 bis 120 bar, bevorzugt 50 bis 100 bar, besonders bevorzugt 60 bis 100 bar und einem Wasserstoffpartialdruck von 10 bis 50 bar, bevorzugt 15 bis 40 bar, besonders bevorzugt 20 bis 35 bar umgesetzt werden. Der Gesamtdruck der Reaktion setzt sich aus dem Dampfdruck der Reaktionspartner und dem Wasserstoffpartialdruck zusammen. Auf den Auslaß von Abgas kann in der Regel weitgehend verzichtet werden, da sich nach dem erfindungsgemäßen Verfahren fremde Inertgase praktisch nicht bilden. Am Ofenende kann das Reaktionsprodukt in einer Vorlage, die standgeregelt betrieben werden kann, kontinuierlich entnommen werden. Die Reaktion ist mit ca. 7 Kcal/mol leicht exotherm. Deshalb ist es leicht, das Reaktionsgefäß isotherm zu halten, indem man einen Teil des Umsetzungsproduktes mittels einer Kreispumpe nach vorhergehender Kühlung auf die Katalysatorschüttung zurückführt. Der Reaktionsaustrag kann noch nicht umgesetztes primäres Amin II und nicht umgesetzten Alkohol enthalten. Beide lassen sich destillativ abtrennen und erneut in die erfindungsgemäße Reaktion einsetzten.

Das Molverhältnis von primärem Amin II zum Alkohol beträgt in der Regel > 1,5, also 1,5 : 1 bis 50 : 1, bevorzugt 1,7 : 1 bis 10 : 1 besonders bevorzugt 2 : 1 bis 5 : 1.

Das Verfahren kann an suspendierten Katalysatoren oder bevorzugt an Festbettkatalysatoren in Sumpffahrweise durchgeführt werden.

An Festbettkatalysatoren arbeitet man zweckmäßig folgendermaßen:
Als Reaktor verwendet man z. B. ein senkrecht angeordnetes zylindrisches Gefäß mit den üblichen Einrichtungen zur Kühlung und Heizung und der Zufuhrmöglichkeit der Reaktionsteilnehmer. Das Reaktionsgefäß kann mit dem Katalysator, der beliebig geformt sein kann, gefüllt werden. Häufig verwendet man den Katalysator als Strangpreßling mit einem Durchmesser von 3 bis 6 mm und einer Länge bis zu 5 cm. Er kann aber auch tablettiert in Form von Zylindern mit den Maßen von z.B. 5 mm Durchmesser und 5 mm Höhe oder als Kugeln oder in jeder anderen beliebigen Form eingesetzt werden.

In der bevorzugten Ausführungsform des erfindungsgemäßen Verfahrens verwendet man Kupferchromit / Bariumchromit-Katalysatoren, deren Bariumgehalt > 1 Gew.-% inbesondere > 3 Gew.-% sein soll. Ein für die Aminierung sehr gut geeigneter Katalysator ist der G 22, der Süd-Chemie, der neben Sauerstoff 33 Gew.-% Kupfer, 27 Gew.-% Chrom und 11 Gew.-% Barium enthält. Wird in den Katalysatoren Barium durch beispielsweise Magnesium oder Kalzium ersetzt, so weisen diese nur ca. 60 % der Aktivität und ca. 90 % der Selektivität der bariumhaltigen Katalysatoren auf. Die Herstellung der Katalysatoren ist aus dem Handbuch "Houben-Weyl, Methoden der organischen Chemie", 4. Auflage, 1955, Georg Thieme Verlag Stuttgart, in Band 4/1, Seite 180 bis 183 bekannt.

Der oben genannte Katalysator G 22 kann in Tablettenform, 3 x 3 und 4,5 x 4,5 mm, verwendet werden. Der Katalysator wird zweckmäßiger Weise vor der Verwendung durch Reduktion mit Wasserstoff bei Temperaturen zwischen 160 und 220°C aktiviert. Bei der nach dem erfindungsgemäßen Verfahren ebenfalls möglichen Suspensionsfahrweise ist der reduzierte Kupferkatalysator in den Reaktionskomponenten Alkohol und sekundärem Amin suspendiert.

Obwohl die Verwendung des Katalysators mit hohem Bariumgehalt bereits eine ausreichende Selektivität liefert, ist es eine wünschenswerte Maßnahme beim vorliegenden Verfahren, den Reaktionspartnern ein Alkalioxid, ein Erdalkalioxid, ein Alkalihydroxid und/oder ein Erdalkalihydroxid zuzugeben. Durch diese Maßnahme gelingt es, Umalkylierungsreaktionen und Disproportionierungen besonders verteilhaft zu unterdrücken. Dabei war es überraschend, daß diese Wirkung verstärkt eintritt, wenn die Alkali- bzw. Erdalkaliverbindung den flüssigen Reaktionspartnern in kontinuierlicher Weise zugeführt wird. Diese Wirkung ist weniger stark ausgeprägt, wenn der Alkaligehalt dem Katalysator-Formkörper zugeführt wird.

Die Reaktionsgeschwindigkeit ist sehr hoch, es lassen sich ohne weiteres 100-300 Gew.-Teile sekundäres Amin I pro Liter Katalysatorvolumen in der Stunde erzeugen.

Die Katalysatoren behalten in der Regel beim erfindungsgemäßen Verfahren über lange Zeiträume (z.B. 2 bis 3 Jahre) ihre Aktivität und Selektivität.

Die Substituenten R¹ und R² in den Verbindungen I und II sowie im Alkohol R²OH haben folgende Bedeutungen:
R¹ und R²
- unabhängig voneinander
- C₁- bis C₁₆-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, n-Nonyl, iso-Nonyl, n-Decyl, iso-Decyl, n-Undecyl, iso-Undecyl, n-Dodecyl und iso-Dodecyl, bevorzugt C₁- bis C₈-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl und iso-Octyl, besonders bevorzugt C₁- bis C₄-Alkyl wie Methyl, Ethyl, n-Propyl, iso-Propyl, n-Butyl, iso-Butyl, sec.-Butyl und tert.-Butyl.

Als Alkohole R²-OH eignen sich insbesondere einwertige aliphatische Alkohole R²-OH z. B. Ethanol, n-Propanol, i-Propanol, n-Butanol oder sec.-Butanol und Hexanol. Als primäre Amine II eignen sich z.B. Methylamin, Ethylamin, Isopropylamin, n-Butylamin, n-Hexylamin.

### Beispiele

Die genannten Teile sind Gew.-Teile, sie verhalten sich zu Vol.-Teilen wie kg zu l.

### Beispiel 1

Für die kontinuierliche Herstellung des Methylethylamins wurde verflüssigtes Monomethylamin technischer Qualität und mit 1,2 % Toluol vergälltes Ethanolazeotrop (95,6 % Ethanol, 4,4 % Wasser) verwendet.

Die Aminierung wurde in einem senkrecht stehenden Reaktionsrohr mit 1000 Vol-Teilen Reaktionsinhalt durchgeführt. Das Verhältnis des Durchmessers zur Länge des Reaktionsrohres betrug 1:40. Mittels eines organischen Wärmeträgers, der in einem Heizmantel umgepumpt wurde, ließ sich das Reaktionsrohr thermostatisieren. In das Reaktionsrohr waren 700 Vol-Teile eines Katalysators gefüllt, der die Form von Zylindern hatte, die 3 mm Höhe und 3 mm Durchmesser maßen. Der Katalysator G 22 (Süd-Chemie AG) war reduziert und aktiviert und vor Aktivitätsverlust durch Dekanolüberstellung geschützt.

Wird der Katalysator in nicht voraktivierter Form verwendet, so kann er auf folgende Weise aktiviert und mit Wasserstoff reduziert werden.

Man rieselt stündlich etwa 300 Vol-Teile Ethanol über den Katalysator und führt diesem von unten nach oben bei 180 bis 200°C zunächst ein Stickstoff-/Wasserstoffgemisch und später reinen Wasserstoff zu. Die Aktivierung des Katalysators ist beendet, wenn im Ethanolazeotrop kein Reduktionswasser vom Katalysator mehr nachweisbar ist.

Zur Umsetzung werden dem Ofen dann bei 200°C stündlich 1000 Vol-Teile Monomethylamin und 500 Vol-Teile Ethanol zugeführt. Im Durchschnitt betrug das Molverhältnis Amin: Ethanol etwa 2,5 : 1. Dem verwendeten Ethanolazeotrop waren 0,05 Gew.-% Natriumhydroxid in Form einer 50 %igen wässrigen Lösung zugefügt worden. Bei 80 bar Gesamtdruck (Wasserstoffpartialdruck ca. 30 bar) werden die Reaktionspartner von unten nach oben zugefahren, so daß der Katalysator ganz mit Sumpfphase überstellt war. Das Reaktionsprodukt wurde in einer Druckvorlage gesammelt und am Kopf der Vorlage stündlich ca. 0,5 bis 2 Vol-Teile Wasserstoff als Abgas gefahren. Das die Vorlage verlassende Reaktionsprodukt (standgeregelt) hatte nach gaschromatographischer Analyse und Destillationsanalyse folgende Zusammensetzung (wasserfrei gerechnet):

| | |
|---|---|
| Undestillierbarer Rückstand: | 0,2 Gew.-% |
| Unbekannte: | 0,3 Gew.-% |
| Methylethylamin: | 15 Gew.-% |
| Dimethylethylamin: | <0,1 Gew.-% |
| Diethylmethylamin: | 1,2 Gew.-% |
| Toluol: | 1 Gew.-% |
| Ethanol: | 35,1 Gew.-% |
| Monomethylamin: | 47,1 Gew.-% |

Durch Destillation unter Druck (2 bar) läßt sich aus dem Rohgemisch Methylethylamin mit über 99,8 % Reinheit gewinnen. Ein ähnliches Ergebnis wird erzielt, wenn dem Ethanol statt Natriumhydroxid Kaliumhydroxid oder Lithiumhydroxid zugesetzt wurde.

### Beispiel 2

Setzt man unter folgenden Reaktionsparametern n-Propylamin mit n-Butanol im Molverhältnis 1,8 : 1 bei 200°C, 80 bar Gesamtdruck und 50 Vol-Teilen Abgas um und fährt stündlich das Gemisch aus Butanol und Propylamin in einer Menge von 500 ml über den Katalysator, so erhält man einen Reaktionsaustrag, der wie folgt zusammengesetzt ist (wasserfrei gerechnet):

| | |
|---|---|
| Undestillierbarer Rückstand: | 0,1 Gew.-% |
| n-Propylamin: | 18,5 Gew.-% |
| n-Butylamin: | 1,8 Gew.-% |
| n-Butanol: | 38,9 Gew.-% |
| Di-n-propylamin: | 5,3 Gew.-% |
| n-Propyl-n-butylamin: | 28,9 Gew.-% |
| Di-n-butylamin: | 2,2 Gew.-% |

### Beispiel 3

Ein Rührbehälter mit einem Reaktionsvolumen von 2000 Teilen wurde mit 690 Teilen wasserfreiem Ethanol, 720 Teilen n-Butylamin und 100 Teilen des Katalysators G 22 (Süd-Chemie), der zuvor gemahlen worden war, beschickt.

Der Kupferkatalysator lag in der aktivierten Form vor. Bei 20°C wurden dem Rührbehälter 20 bar Wasserstoff aufgepreßt und anschließend auf 210°C aufgeheizt. Der Gesamtdruck des Reaktionssystems stieg dabei auf ca. 60 bar an. Man rührte das System 6 Stunden lang bei Reaktionstemperatur, ließ dann abkühlen, den Katalysator sich absetzen und drückte die überstehende klare Lösung ab. Sie hatte etwa folgende Zusammensetzung (wasserfrei gerechnet):

| | |
|---|---|
| Hochsieder: | 0,1 Gew.-% |
| Unbekannte: | 0,4 Gew.-% |
| Ethanol: | 11,2 Gew.-% |
| n-Butylamin: | 40,9 Gew.-% |
| Diethyl-n-butylamin: | 10,3 Gew.-% |
| Ethyl-n-butylamin: | 37,2 Gew.-% |

## Patentansprüche

1. Verfahren zur Herstellung von Dialkylaminen der allgemeinen Formel I 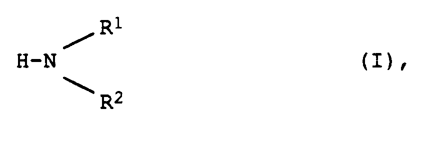 in der
R¹, R² C₁- bis C₁₆-Alkyl bedeuten, durch Umsetzung von Monoalkylaminen der allgemeinen Formel II 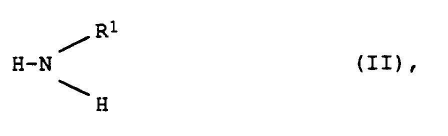 in der R¹ C₁- bis C₁₆-Alkyl bedeutet, mit Alkoholen R²-OH in Gegenwart eines Kupferchromit-Erdalkalichromit enthaltenden Hydrierungs-/Dehydrierungskatalysators, dadurch gekennzeichnet, daß man die Umsetzung in flüssiger Phase im Molverhältnis Amin zum Alkohol von 1,5 : 1 bis 50 : 1 in Anwesenheit des bei der Reaktion gebildeten Wassers bei Temperaturen von 180 bis 210°C und Drücken von 40 bis 120 bar durchführt.

2. Verfahren zur Herstellung von Dialkylaminen I nach Anspruch 1, dadurch gekennzeichnet, daß man die Umsetzung in Gegenwart von Wasserstoff durchführt.

3. Verfahren zur Herstellung von Dialkylaminen I nach Anspruch 1, dadurch gekennzeichnet, daß man die Umsetzung bei einem Wasserstoffpartialdruck von 10 bis 50 bar durchführt.

4. Verfahren zur Herstellung von Dialkylaminen I nach Anspruch 1, dadurch gekennzeichnet, daß man die Umsetzung in Gegenwart von 0,01 bis 2 Gew.-% eines Alkalioxids, eines Erdalkalioxids, eines Alkalihydroxids und/oder Erdalkalihydroxids durchführt.
